(19) Europäisches Patentamt
European Patent Office
Office européen des brevets

(11) Veröffentlichungsnummer: **0 208 264**
**B1**

(12) # EUROPÄISCHE PATENTSCHRIFT

(45) Veröffentlichungstag der Patentschrift: **23.08.89**

(21) Anmeldenummer: **86109073.6**

(22) Anmeldetag: **03.07.86**

(51) Int. Cl.⁴: **C 07 D 498/10,** C 07 D 519/00, C 08 K 5/35, C 09 D 7/12, C 09 K 15/20 // (C07D498/10, 263:00, 221:00)

(54) **1-Oxa-3-oxo-4,8-diaza-spiro- 4,5 decan-Verbindungen.**

(30) Priorität: **10.07.85 DE 3524542**

(43) Veröffentlichungstag der Anmeldung: **14.01.87 Patentblatt 87/3**

(45) Bekanntmachung des Hinweises auf die Patenterteilung: **23.08.89 Patentblatt 89/34**

(84) Benannte Vertragsstaaten: **AT BE CH DE FR GB IT LI NL SE**

(56) Entgegenhaltungen:
**EP-A-0 094 605**
**EP-A-0 095 076**
**DE-A-2 063 573**
**DE-A-2 738 340**
**DE-A-2 933 732**
**DE-A-3 149 453**
**DE-A-3 408 949**
**US-A-4 314 933**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Ertl, Josef, Dr., Eichenstrasse 14, D-8857 Wertingen (DE)**

EP 0 208 264 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft 1-Oxa-3-oxo-4,8-diaza-spiro-[4,5]-decan-Verbindungen, die als Lichtschutzmittel für Polymere oder als Zwischenprodukte für die Herstellung von Kunststoffadditiven verwendet werden können, sowie ein Verfahren zu ihrer Herstellung.

Verbindungen der Formel

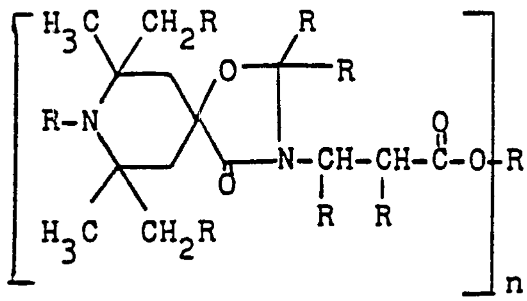

sind bekannt (vgl. Deutsche Offenlegungsschrift 3 149 453). Allerdings ist das Verfahren zu ihrer Herstellung kompliziert, da während der Reaktion das Reaktionsmedium mehrfach gewechselt wird, was zusätzliche Extraktionen und Destillationen bedingt.

Es wurden nun neue 1-Oxa-3-oxo-4,8-diaza-spiro-[4,5]-decan-Verbindungen gefunden.

Gegenstand der Erfindung sind somit 1-Oxa-3-oxo-4,8-diaza-spiro-[4,5]decan-Verbindungen der Formel I sowie ein Verfahren zu ihrer vereinfachten Herstellung.

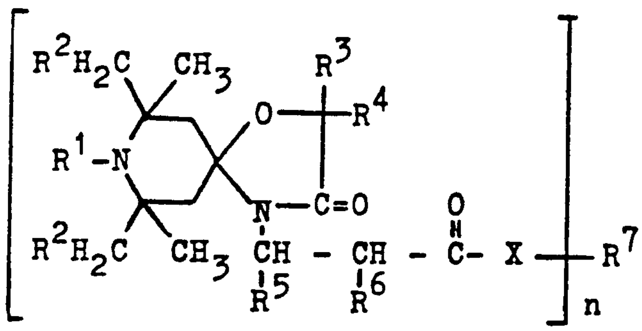

(I)

worin

n eine ganze Zahl von 1 bis 4 ist,
Wasserstoff, $C_1$-$C_4$-Alkyl, Benzyl, Allyl, $C_2$-$C_{30}$-Alkanoyl, $C_3$-$C_{20}$-Alkenoyl, $C_7$-$C_{11}$-Aroyl, $C_8$-$C_{14}$-Arylalkanoyl oder $C_8$-$C_{20}$-Alkylaryl ist.
$R^2$ Wasserstoff oder $C_1$-$C_4$-Alkyl bedeutet,
$R^3$ Wasserstoff, $C_1$-$C_{18}$-Alkyl, $C_5$-$C_{12}$-Cycloalkyl, eine Phenyl- oder Naphthylgruppe, die durch Chlor oder $C_1$-$C_4$-Alkyl substituiert sein kann, oder eine gegebenenfalls durch $C_1$-$C_4$-Alkyl substituierte $C_7$-$C_{12}$-Phenylalkylengruppe ist,
$R^4$ Wasserstoff, $C_1$-$C_4$-Alkyl, $C_5$-$C_{12}$-Cycloalkyl, durch -COOH, Carb-$C_1$-$C_4$-alkoxi oder Carbamoyl substituiertes $C_1$-$C_3$-Alkenyl, eine Phenyl-, Naphthyl- oder Pyridylgruppe, die durch $C_1$-$C_4$-Alkoxi oder $C_1$-$C_4$-Alkyl substituiert sein kann, oder eine $C_7$-$C_{12}$-Phenylalkylgruppe, die durch $C_1$-$C_4$-Alkyl substituiert sein kann, bedeutet, oder
$R^3$ und $R^4$ zusammen mit dem sie bindendem Kohlenstoffatom eine $C_5$-$C_{12}$-Cycloalkylgruppe, die durch eine bis vier $C_1$-$C_4$-Alkylgruppen substituiert sein kann, oder einen Rest der Formel II

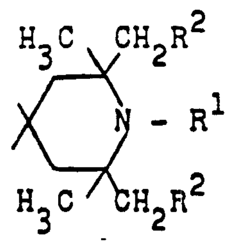

(II),

worin
$R^1$ und $R^2$ die obengenannte Bedeutung haben, bilden,

$R^5$ Wasserstoff, Methyl, Phenyl oder Carb-$C_1$-$C_{21}$-alkoxi ist,

$R^6$ Wasserstoff oder Methyl bedeutet,

$R^7$ bei n = 1
Wasserstoff, $C_1$-$C_{21}$-Alkyl, $C_2$-$C_{22}$-Alkenyl, $C_7$-$C_{18}$-Phenylalkyl, $C_5$-$C_{12}$-Cycloalkyl, Phenyl, Naphthyl, $C_7$-$C_{18}$-Alkylphenyl, ein Rest der Formel

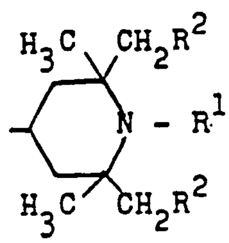

worin $R^1$ und $R^2$ die obige Bedeutung haben, oder $C_2$-$C_{20}$-Alkyl, welches unterbrochen sein kann durch -O- oder

-N- und/oder substituiert durch einen Rest der Formel III
|
$R^8$

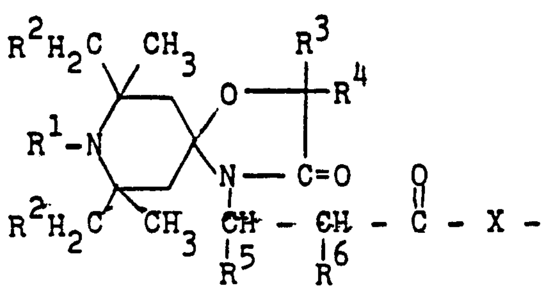

(III),

Hoder durch $C_1$-$C_{21}$-Alkylcarboxyl, wobei $R^1$, $R^2$, $R^3$, $R^4$, $R^5$ und $R^6$ die obige Bedeutung haben und $R^8$ Wasserstoff, $C_1$-$C_{10}$-Alkyl oder ein Rest der Formel

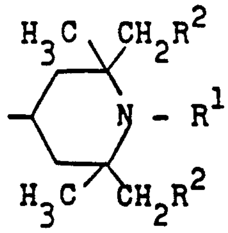

ist
worin $R^1$ und $R^2$ die obige Bedeutung habe,
ist,

$R^7$ bei n = 2
geradkettiges oder verzweigtes $C_1$-$C_{30}$-Alkylen, $C_2$-$C_{30}$-Alkenylen, Phenyldialkylen, wobei diese Reste durch -O- oder
-N-,
|
$R^8$
worin $R^8$ die obige Bedeutung hat, unterbrochen sein können, bedeutet,

$R^7$ bei n = 3 oder 4
einen Rest der Formeln IV, V, VI oder VII

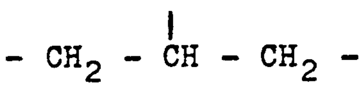
(IV),

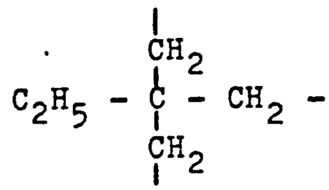
(V),

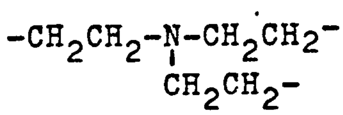
(VI),

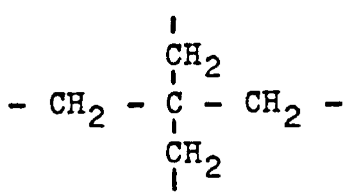
(VII) bedeutet, und

X -O- oder
$R^8$
|
-N-,
worin $R^8$ die obige Bedeutung hat, ist.

n ist vorzugsweise 1 oder 2.

$R^1$ ist bevorzugt Wasserstoff, $C_1$-$C_4$-Alkyl, $C_2$-$C_{18}$-Alkanoyl, beispielsweise Methyl, Ethyl, Propyl, Butyl, Acetyl, Propionyl, Butyryl, Lauroyl, Stearoyl, besonders bevorzugt Wasserstoff oder einer der genannten Säurereste. Insbesondere ist $R^1$ Wasserstoff.

$R^2$ ist bevorzugt Wasserstoff oder $C_1$-$C_4$-Alkyl, beispielsweise Methyl, Ethyl, Propyl, Butyl. Insbesondere ist $R^2$ Wasserstoff.

$R^3$ und $R^4$ sind unabhängig voneinander $C_1$-$C_{18}$-Alkyl, $C_5$-$C_{12}$-Cycloalkyl oder Phenyl, beispielsweise Ethyl, Butyl, Octyl, Lauryl, Stearyl, Cyclohexyl, Cyclodecyl, besonders bevorzugt $C_1$-$C_7$-Alkyl. Insbesondere sind $R^3$ und $R^4$ $C_1$-$C_4$-Alkyl, beispielsweise Methyl.

$R^3$ und $R^4$ zusammen mit dem sie bindenden Kohlenstoffatom sind bevorzugt $C_5$-$C_{12}$-Cycloalkylen, besonders bevorzugt $C_6$- bzw. $C_{12}$-Cycloalkylen, insbesondere Cyclododecylen.

$R^5$ ist bevorzugt Wasserstoff, Methyl oder Phenyl, besonders bevorzugt Wasserstoff.

$R^6$ ist bevorzugt Wasserstoff oder Methyl. Insbesondere ist $R^6$ Wasserstoff.

$R^7$ ist bevorzugt $C_1$-$C_{21}$-Alkyl, geradkettiges oder verzweigtes $C_1$-$C_{30}$-Alkylen, beispielsweise Methyl, Butyl, Octyl, Lauryl, Myristyl, Stearyl, Ethyliden, Butyliden, Hexyliden, besonders bevorzugt $C_1$-$C_{15}$-Alkyl oder $C_2$-$C_6$-Alkylen. Insbesondere ist $R^7$ $C_{12}$-$C_{14}$-Alkyl, beispielsweise Lauryl oder $C_2$- bzw. $C_6$-Alkylen, beispielsweise Hexylen.

$R^8$ ist bevorzugt Wasserstoff oder 2,2,6,6-Tetraalkylpiperid-4-yl, insbesondere 2,2,6,6-Tetramethylpiperid-4-yl.

Die Verbindungen der Formel I entstehen durch Umsetzung von Verbindungen der Formel VIII

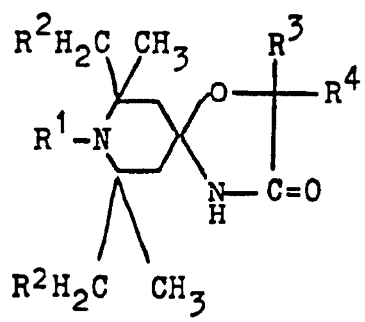

(VIII),

mit Verbindungen der Formel IX

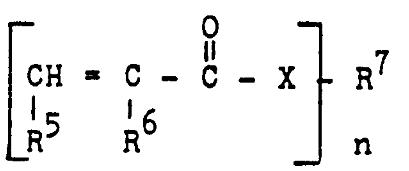

(IX),

wobei n, $R^1$, $R^2$, $R^3$, $R^4$, $R^5$, $R^6$ und $R^7$ und X die obengenannte Bedeutung haben, in einem inerten Lösemittel bei einer Temperatur von 30 bis 150° C in Gegenwart eines basischen Katalysators, wobei

die Umsetzung in Gegenwart von 0,05 bis 20 Mol-%, bezogen auf die Verbindung VIII, eines Phasentransferkatalysators in einem bei Raumtemperatur flüssigen aromatischen Kohlenwasserstoff durchgeführt wird.

Die Verbindungen der Formel VIII können beispielsweise gewonnen werden, indem in einem organischen Lösungsmittel bei einer Temperatur von 20 bis 100° C bei Anwesenheit eines wasserentziehenden Mittels ein 2,6-Dimethyl-2,6-dialkylpiperidon der Formel

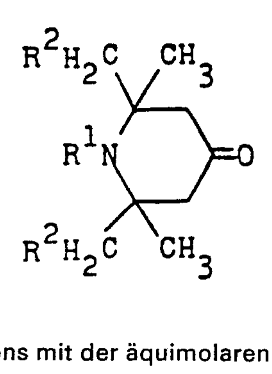

oder ein Salz eines derartigen Piperidons mit der äquimolaren Menge eines α-Hydroxamids der Formel

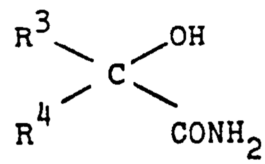

oder mit der äquimolaren Menge eines Cyanhydrins der Formel

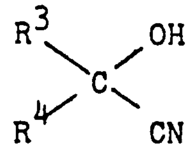

umgesetzt wird. Die Herstellung ist literaturbekannt.

Geeignete Verbindungen der Formel VIII sind beispielsweise:

2,7,7,9,9-Pentamethyl-1-oxa-3-oxo-4,8-diaza-spiro-[4,5]-decan
2-Ethyl-7,7,9,9-tetramethyl-1-oxa-3-oxo-4,8-diaza-spiro-[4,5]-decan
2-Propyl-7,7,9,9-tetramethyl-1-oxa-3-oxo-4,8-diaza-spiro-[4,5]-decan
2-Butyl-7,7,9,9-tetramethyl-1-oxa-3-oxo-4,8-diaza-spiro-[4,5]-decan
2-iso-Butyl-7,7,9,9-tetramethyl-1-oxa-3-oxo-4,8-diaza-spiro-[4,5]-decan
2-Pentyl-7,7,9,9-tetramethyl-1-oxa-3-oxo-4,8-diaza-spiro-[4,5]-decan
2-iso-Pentyl-7,7,9,9-tetramethyl-1-oxa-3-oxo-4,8-diaza-spiro-[4,5]-decan
2-iso-Heptyl-7,7,9,9-tetramethyl-1-oxa-3-oxo-4,8-diaza-spiro-[4,5]-decan
2-Phenyl-7,7,9,9-tetramethyl-1-oxa-3-oxo-4,8-diaza-spiro-[4,5]-decan
2,2,7,7,9,9-Hexamethyl-1-oxa-3-oxo-4,8-diaza-spiro-[4,5]-decan
2,2,7,7,8,9,9-Heptamethyl-1-oxa-3-oxo-4,8-diaza-spiro-[4,5]-decan
2,2-Diethyl-7,7,9,9-tetramethyl-1-oxa-3-oxo-4,8-diaza-spiro-[4,5]-decan
2,2-Diethyl-7,7,8,9,9-pentamethyl-1-oxa-3-oxo-4,8-diaza-spiro-[4,5]-decan
2,2-Dipropyl-7,7,9,9-tetramethyl-1-oxa-3-oxo-diaza-spiro-[4,5]-decan
2,2-Dibutyl-7,7,9,9-tetramethyl-1-oxa-3-oxo-4,8-diaza-spiro-[4,5]-decan
2,2-Dipentyl-7,7,9,9-tetramethyl-1-oxa-3-oxo-4,8-diaza-spiro-[4,5]-decan
2-Ethyl-2,7,7,9,9-pentamethyl-1-oxa-3-oxo-4,8-diaza-spiro-[4,5]-decan
2-Propyl-2,7,7,9,9-pentamethyl-1-oxa-3-oxo-4,8-diaza-spiro-[4,5]-decan
2-iso-Propyl-2,7,7,9,9-pentamethyl-1-oxa-3-oxo-4,8-diaza-spiro-[4,5]-decan
2-Butyl-2,7,7,9,9-pentamethyl-1-oxa-3-oxo-4,8-diaza-spiro-[4,5]-decan
2-iso-Butyl-2,7,7,9,9-pentamethyl-1-oxa-3-oxo-4,8-diaza-spiro-[4,5]-decan
2-Pentyl-2,7,7,9,9-pentamethyl-1-oxa-3-oxo-4,8-diaza-spiro-[4,5]-decan
2-iso-Pentyl-2,7,7,9,9-pentamethyl-1-oxa-3-oxo-4,8-diaza-spiro-[4,5]-decan
2-Hexyl-2,7,7,9,9-pentamethyl-1-oxa-3-oxo-4,8-diaza-spiro-[4,5]-decan
2-Heptyl-2,7,7,9,9-pentamethyl-1-oxa-3-oxo-4,8-diaza-spiro-[4,5]-decan
2-Nonyl-2,7,7,9,9-pentamethyl-1-oxa-3-oxo-4,8-diaza-spiro-[4,5]-decan
2-Undecyl-2,7,7,9,9-pentamethyl-1-oxa-3-oxo-4,8-diaza-spiro-[4,5]-decan
2-Ethyl-2-butyl-7,7,9,9-tetramethyl-1-oxa-3-oxo-4,8-diaza-spiro-[4,5]-decan
2-Ethyl-2-pentyl-7,7,9,9-tetramethyl-1-oxa-3-oxo-4,8-diaza-spiro-[4,5]-decan
2-Ethyl-2-iso-pentyl-7,7,9,9-tetramethyl-1-oxa-3-oxo-4,8-diaza-spiro-[4,5]-decan
2,2,7,7,9,9-Hexamethyl-1-oxa-3-oxo-4,8-diaza-8-acetyl-spiro-[4,5]-decan
2,2-Diethyl-7,7,9,9-tetramethyl-1-oxa-3-oxo-4,8-diaza-8-acetyl-spiro-[4,5]-decan
2,2,4,4-Tetramethyl-7-oxa-13-oxo-3,14-diaza-dispiro-[5,1,4,2]-tetradecan
2,2,4,4-Tetramethyl-7-oxa-14-oxo-3,15-diaza-dispiro-[5,1,5,2]-pentadecan
2,2,4,4-Tetramethyl-7-oxa-20-oxo-3,21-diaza-dispiro-[5,1,11,2]-heneicosan

Geeignete Verbindungen der Formel IX sind beispielsweise:

Acrylsäuremethylester
Acrylsäureethylester
Acrylsäure-n-butylester
Acrylsäure-iso-butylester
Acrylsäure-tert.-butylester
Acrylsäure-2-ethylhexylester

Acrylsäureoctylester
Acrylsäurelaurylester
Acrylsäuremyristylester
Acrylsäure-2-diethylaminoethylester

Methacrylsäuremethylester
Methacrylsäureethylester
Methacrylsäure-n-butylester
Methacrylsäure-iso-butylester
Methacrylsäure-tert.-butylester
Methacrylsäurelaurylester
Methacrylsäurecyclohexylester
Methacrylsäureallylester
Methacrylsäure-2-ethoxyethylester
Methacrylsäure-2-dimethylaminoethylester

Crotonsäuremethylester
Crotonsäureethylester

1,4-Butandioldiacrylat
1,6-Hexandioldiacrylat
2-Ethyl-2-hydroxymethyl-1,3-propandiol-triacrylat
1,4-Butandioldimethacrylat

Acrylsäureamid
N,N'-Methylen-bis-(acrylsäureamid)
N,N'-Ethylen-bis-(acrylsäureamid)
N,N'-Hexamethylen-bis-(acrylsäureamid)

Glyoxal-bis(acrylsäureamid)

Acrylsäure-2,2,6,6-tetramethylpiperid-4-yl-ester
Crotonsäure-2,2,6,6-tetramethylpiperid-4-yl-ester
Methacrylsäure-2,2,6,6-tetramethylpiperid-4-yl-ester

Acrylsäure-2,2,6,6-tetramethylpiperid-4-yl-amid
Crotonsäure-2,2,6,6-tetramethylpiperid-4-yl-amid
Methacrylsäure-2,2,6,6-tetramethylpiperid-4-yl-amid

N,N'-Bis-(2,2,6,6-tetramethylpiperid-4-yl)-N,N'-bis-(acrylsäureamid)
N,N'-Bis-(2,2,6,6-tetramethylpiperid-4-yl)-N,N'-hexamethylen-bis-(acrylsäureamid)

Eine weitere Herstellungsmöglichkeit ist die Synthese der Verbindungen der Formel I mit X = -O- und n = 1 und die anschließende Umsetzung mit Aminen der Formel

$R^7$-$[NHR^8]_n$

Für das erfindungsgemäße Verfahren wird als Lösemittel ein bei Raumtemperatur flüssiger aromatischer Kohlenwasserstoff verwendet, vorzugsweise Toluol oder Xylol.

Als Phasentransferkatalysator wird vorzugsweise zugesetzt ein Polyethylenglykoldialkylether, ein substituiertes Phosphoniumsalz, beispielsweise Tetraalkylphosphoniumhalogenid oder ein substituiertes Ammoniumsalz, beispielsweise Tetraalkylammoniumhalogenid oder Trialkylbenzylammoniumhalogenid. Insbesondere wird Triethylbenzylammoniumchlorid oder ein Tetraalkylphosphoniumbromid zugesetzt. Die Menge beträgt 0,05 bis 20, vorzugsweise 0,1 bis 10, insbesondere 1 bis 10 Mol-%, bezogen auf die Verbindung der Formel VIII.

Die Verbindung IX wird in einer Menge von 1/n bis 10/n, vorzugsweise 1/n bis 3/n, insbesondere 1/n bis 1,5/n Mol, bezogen auf 1 Mol der Verbindung VIII, eingesetzt. n hat die oben angegebene Bedeutung.

Die Reaktionstemperatur beträgt 30 bis 150, vorzugsweise 50 bis 120, insbesondere 70 bis 120°C.

Die Reaktion wird in Gegenwart eines basischen Katalysators durchgeführt. Als solcher dient ein Alkalimetall, vorzugsweise Natrium, welches in einer Menge von 1 bis 30 Mol-%, vorzugsweise 2 bis 10 Mol-%, bezogen auf die Verbindung VIII, verwendet wird.

Das erfindungsgemäße Verfahren bringt gegenüber dem Stand der Technik erhebliche Vorteile. Zunächst wird nur ein einziges, technisch einfach zu handhabendes Lösemittel bzw. Lösemittelgemisch verwendet. Überraschenderweise wird durch den Phasentransferkatalysator bewirkt, daß die Reaktion wesentlich schneller und vor allem vollständiger abläuft, so daß die Verbindung IX nicht mehr im vielfachen Überschuß eingesetzt werden muß, sondern ein geringer Überschuß genügt. Trotzdem wird eine höhere Ausbeute

erhalten und die Menge der Nebenprodukte verringert.

Die erfindungsgemäßen Verbindungen der Formel (I) werden vor allem als Lichtstabilisatoren eingesetzt, beispielsweise für Polyolefine, insbesondere Polyethylen und Polypropylen, Ethylen/Propylen-Copolymere, Polybutylen, sowie Polystyrol, chloriertes Polyethylen, sowie Polyvinylchlorid, Polyester, Polycarbonat, Polymethylmethacrylate, Polyphenylenoxide, Polyamide, Polyurethane, Polypropylenoxid, Polyacetale, Phenol-Formaldehydharze, Epoxidharze, Polyacrylnitril und entsprechende Copolymerisate sowie ABS-Terpolymere. Vorzugsweise verwendet man die erfindungsgemäß hergestellten Verbindungen zum Stabilisieren von Polypropylen, niedermolekularen und hochmolekularen Polyethylen, Ethylen-Propylen-Copolymeren, Polyvinylchlorid, Polyester, Polyamid, Polyurethanen, Polyacrylnitril, ABS, Terpolymeren von Acrylester, Styrol und Acrylnitril, Copolymeren von Styrol und Acrylnitril oder Styrol und Butadien, insbesondere für Polypropylen, Polyethylen, Ethylen-Propylen-Copolymer oder ABS.

Die erfindungsgemäßen Verbindungen können auch für die Stabilisierung von Naturstoffen, beispielsweise Kautschuk, verwendet werden, sowie auch für Schmieröle. Weiterhin eignen sie sich auch für die Stabilisierung von Lacken.

Als Lacke kommen alle in der Industrielackierung verwendeten Arten, vorzugsweise Einbrennlacke, infrage.

Letztere werden bei höherer Temperatur eingebrannt, um optimale Eigenschaften zu erhalten. Bevorzugt sind Naßlacke, welche als Bindemittel enthalten: Kombinationen aus ölmodifizierten Polyesterharzen (Ölalkydharze) und Melamin-Formaldehyd-Harzen oder Kombinationen aus fremdvernetzenden Polyacrylatharzen und Melamin-Formaldehyd-Harzen oder Kombinationen aus gesättigten Polyestern und Melamin-Formaldehyd-Harzen oder selbstvernetzende Polyacrylatharze oder auch Polyacrylatharze mit einpolymerisiertem Styrol. Weiterhin sind zu nennen Zweikomponenten-Acrylatharzlacke, bestehend aus hydroxylgruppenhaltigem Acrylatharz und aliphatischen oder aromatischen Isocyanaten, wie auch thermoplastische Polyacrylatharzlacke. Zu nennen sind auch Zweikomponenten-Polyurethanharzlacke, bestehend aus hydroxylgruppenhaltigen Polyesterharzen und/oder Polyätherharzen, gehärtet mit aliphatischen oder aromatischen Isocyanaten. Für Metallise-Lacke haben vor allem Bedeutung die thermoplastischen Polyacrylatharze oder fremdvernetzenden Polyacrylatharze in Kombination mit Butanol-veretherten Melaminharzen sowie auch hydroxylgruppenhaltige Polyacrylatharze, gehärtet mit aliphatischen Isocyanaten. Ebenso kommen die an sich bekannten Pulverlacke infrage, welche z. B. mit einer Lösung der erfindungsgemäßen Verbindungen behandelt wurden.

Das Einbringen der erfindungsgemäßen Verbindungen in die zu schützenden Materialien erfolgt nach an sich bekannten Methoden, wobei auch Monomere oder Vorpolymerisate bzw. Vorkondensate mit diesen Stabilisatoren versehen werden können.

Neben den Verbindungen der Formel (I) können den Kunststoffen noch weitere Stabilisatoren zugesetzt werden. Derartige weitere Verbindungen sind z. B. Antioxidantien auf der Basis sterisch gehinderter Phenole, oder Schwefel oder Phosphor enthaltende Co-stabilisatoren oder eine Mischung von geeigneten sterisch gehinderten Phenolen und Schwefel und/oder Phosphor enthaltenden Verbindungen. Solche Verbindungen sind z. B. Benzofuranon(2)- und/oder Indolinon(2)verbindungen, sterisch gehinderte Phenole wie β-(4-Hydroxy-3,5-di-t-butylphenyl)-propionsäurestearylester, Tetrakis-[methylen-3(3',5'-di-t-butyl-4-hydroxyphenyl)-propionat]-methan, 1,3,3-Tris-(2-methyl-4-hydroxy 5-t-butylphenyl)-butan, 1,3,5 Tris-(4-t-butyl-3-hydroxy-2,6-dimethylbenzyl)-1,3,5-triazin-2,4,6-(1H, 3H, 5H)-trion, Bis-(4-t-butyl-3-hydroxy-2,6-dimethylbenzyl)-dithiolterephthalat, Tris-(3,5-t-butyl-4-hydroxybenzyl) isocyanurat, Triester der β-(4-Hydroxy-3,5-di-t-butylphenyl)-propionsäure mit 1,3,5-Tris-(2-hydroxyethyl)-s-triazin 2,4,6-(1H, 3H, 5H)-trion, Bis-[3,3-bis-(4'-hydroxy-3-t-butylphenyl)-butansäure]-glycolester, 2,3,5-Trimethyl-2,4,6-tris-(3,5-di-t-butyl-4-hydroxybenzyl)-benzol, 2,2'-Methylen-bis-(4-methyl-6-t-butylphenyl)-terephthalat, 4,4-Methylen-bis-(2,6-di-t-butylphenol), 4,4'-Butyliden-bis-(t-butyl-meta-kresol), 4,4-Thio-bis-(2-t-butyl-5-methyl-phenol), 2,2'-Methylen-bis-(4-methyl-6-t-butyl-phenol). Auch antioxidativ wirkende Co-Stabilisatoren können zugegeben werden, wie z. B. schwefelhaltige Verbindungen, z. B. Distearylthiodipropionat, Dilaurylthiodipropionat, Tetrakis-(methylen-3-hexyl-thiopropionat)-methan, Tetrakis-(methylen-3-dodecyl-thiopropionat)-methan und Dioctadecyldisulfid oder phosphorhaltige Verbindungen wie z. B. Trinonylphenylphosphit; 4,9-Distearyl-3,5,8,10-tetraoxadiphosphaspiroundecan, Tris-(2,4-t-butylphenyl)-phosphit oder Tetrakis-(2,4 di-t-butylphenyl)-4,4'-biphenylylen-diphosphonit.

Die Verbindungen der Formel I und deren oben erwähnte Mischungen kann man auch in Gegenwart weiterer Additive verwenden.

Solche sind an sich bekannt und gehören z. B. zur Gruppe der Aminoacrylverbindungen, der UV-Absorber und Lichtschutzmittel wie die 2-(2'-Hydroxyphenyl)-benztriazole, 2-Hydroxybenzophenone, 1,3-Bis-(2'-hydroxybenzoyl)-benzole, Salicylate, Zimtsäureester, Ester von gegebenenfalls substituierten Benzoesäuren, sterisch gehinderte Amine, Oxalsäurediamide.

Die Anwendungsmenge der erfindungsgemäß hergestellten Verbindungen der Formel I beträgt 0,01 - 5 Gew.-% bei Kunststoffen, 20 bis 80 Gew.-% bei Stabilisatorkonzentraten und 0,02 - 5 Gew.-% bei Lacken.

**BEISPIEL 1**

In 100 g trockenem Toluol wurden 24,0 g (0,1 Mol) 2,2,7,7,9,9-Hexamethyl-1-oxa-3-oxo-4,8-diaza-spiro-[4,5]-decan (Edukt I), 0,8 g Triethylbenzylammoniumchlorid und 0,2 g Natrium zusammengegeben und auf 80° C erhitzt. Innerhalb von 30 Minuten wurden 12,9 g [0,15 Mol] Acrylsäuremethylester zugetropft. Bei 80° C wurde 6

Stunden weitergerührt, anschließend der Ansatz dreimal mit je 50 ml Wasser ausgeschüttelt, die organische Phase über $Na_2SO_4$ getrocknet, filtriert und das Toluol abdestilliert. Zurück blieben 31,6 g weißer Feststoff (98 % d. Theorie) mit einem Schmelzpunkt von 75°C. Dieses Produkt enthielt laut Gaschromatographie (GC) 0,2 Gew.-% 2,2,7,7,9,9-Hexamethyl-1-oxa-3-oxo-4,8-diaza-spiro-[4,5]-decan, aber keinen Acrylsäuremethylester.

**BEISPIEL 2**

Die Durchführung erfolgte wie bei Beispiel 1, jedoch wurden 38,3 g (0,15 Mol) Acrylsäurelaurylester (technisches Gemisch von ca. 53 - 55 % $C_{12}$-Ester und ca. 42 - 45 % $C_{14}$-Ester) anstelle des Acrylsäuremethylesters eingesetzt. Es wurden 52 g einer Öligen Substanz erhalten, die nach GC noch 0,06 Gew.-% Edukt I enthielt.

**BEISPIEL 3**

Wie in Beispiel 1 beschrieben wurden 24,0 g (0,1 Mol) Edukt I mit 10,4 g [0,035 Mol] Trimethylolpropantriacrylat imgesetzt. Es wurden 27,9 g Feststoff mit einem Fp von 80 - 90°C erhalten.

**BEISPIEL 4**

32,6 g (0,1 Mol) der nach Beispiel 1 hergestellten Verbindung wurden in 100 g Xylol gelöst, am Wasserabscheider getrocknet und 5,8 g (0,05 Mol) Hexamethylendiamin und 0,1 g Natriumhydrid zugegeben. In 20 Stunden wurden ca. 3,2 g (0,1 Mol) Methanol über eine Kolonne abdestilliert. Nach dem Abkühlen wurde der Niederschlag abgesaugt und mit siedendem Heptan gewaschen. Das farblose Produkt hatte einen Schmelzpunkt von 20°C.

**BEISPIEL 5**

In 100,0 g Toluol wurden 8,6 g (0,036 Mol) 2,2,7,7,9,9-Hexamethyl-1-oxa-3-oxo-4,8-diaza-spiro-[4,5]-decan vorgelegt und durch Kochen am Wasserabscheider getrocknet. Dann wurden bei 80°C 0,4 g Triethylbenzylammoniumchlorid, 0,2 g Natrium und 4,0 g (0,018 Mol) N,N'-Hexamethylen-bis-(acrylsäureamid) zugegeben und bei dieser Temperatur ca. 12 h gerührt. Der aufgetretene Niederschlag wurde abgesaugt und aus Toluol umkristallisiert. Man erhielt 10.5 g farbloses Produkt: Fp = 204°C; Molmasse gemessen 710, berechnet 704.

| | | | | |
|---|---|---|---|---|
| CHN-Analyse: | gemessen | C 64,7 % | H 9,8 % | N 11,1 % |
| | berechnet | C 64,8 % | H 9,7 % | N 11,9 % |

**Patentansprüche** für die Vertragsstaten: BE, CH, DE, FR, GB, IT, LT, NL, SE

1. 1-Oxa-3-oxo-4,8-diaza-spiro-[4,5]-decan-Verbindungen der Formel I

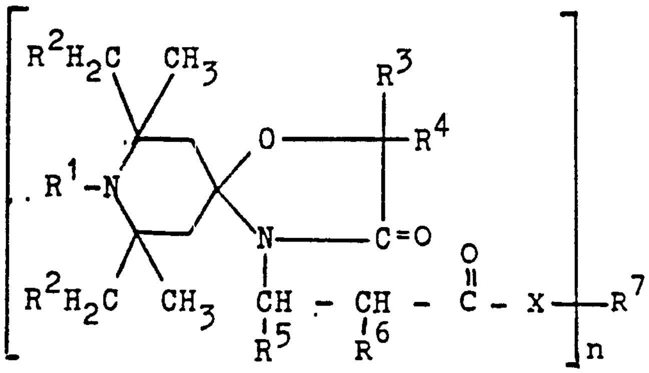

(I),

worin

n eine ganze Zahl von 1 bis 4 ist,

$R^1$ Wasserstoff, $C_1$-$C_4$-Alkyl, Benzyl, Allyl, $C_2$-$C_{30}$-Alkanoyl, $C_3$-$C_{20}$-Alkenoyl, $C_7$-$C_{11}$-Aroyl, $C_8$-$C_{14}$-Arylalkanoyl oder $C_8$-$C_{20}$-Alkylaryl ist,

$R^2$ Wasserstoff oder $C_1$-$C_4$-Alkyl bedeutet,

$R^3$ Wasserstoff, $C_1$-$C_{18}$-Alkyl, $C_5$-$C_{12}$-Cycloalkyl, eine Phenyl- oder Naphthylgruppe, die durch Chlor oder $C_1$-$C_4$-Alkyl substituiert sein kann, oder eine gegebenenfalls durch $C_1$-$C_4$-Alkyl substituierte $C_7$-$C_{12}$-Phenylalkylengruppe ist,

$R^4$ Wasserstoff, $C_1$-$C_4$-Alkyl, $C_5$-$C_{12}$-Cycloalkyl, durch -COOH, Carb-$C_1$-$C_4$-alkoxi oder Carbamoyl substituiertes $C_1$-$C_3$-Alkenyl, eine Phenyl-, Naphthyl- oder Pyridylgruppe, die durch $C_1$-$C_4$-Alkoxi oder $C_1$-$C_4$-Alkyl substituiert sein kann, oder eine $C_7$-$C_{12}$-Phenylalkylgruppe, die durch $C_1$-$C_4$-Alkyl substituiert sein kann, bedeutet, oder

$R^3$ und $R^4$ zusammen mit dem sie bindendem Kohlenstoffatom eine $C_5$-$C_{12}$-Cycloalkylgruppe, die durch eine bis vier $C_1$-$C_4$-Alkylgruppen substituiert sein kann, oder einen Rest der Formel II

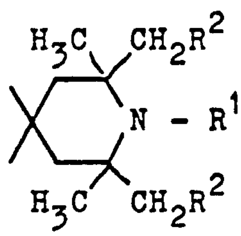

(II),

worin $R^1$ und $R^2$ die obengenannte Bedeutung haben, bilden

$R^5$ Wasserstoff, Methyl, Phenyl oder Carb-$C_1$-$C_{21}$-alkoxi ist,

$R^6$ Wasserstoff oder Methyl bedeutet,

$R^7$ bei n = 1

Wasserstoff, $C_1$-$C_{21}$-Alkyl, $C_2$-$C_{22}$-Alkenyl, $C_7$-$C_{18}$-Phenylalkyl, $C_5$-$C_{12}$-Cycloalkyl, Phenyl, Naphthyl, $C_7$-$C_{18}$-Alkylphenyl, den Rest der Formel

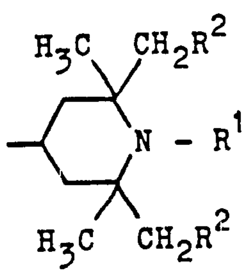

,

worin $R^1$ und $R^2$ die obige Bedeutung haben, $C_2$-$C_{20}$-Alkyl, welches unterbrochen sein kann durch -O- oder
-N- und/oder substituiert
|
$R^8$

durch einen Rest der Formel III

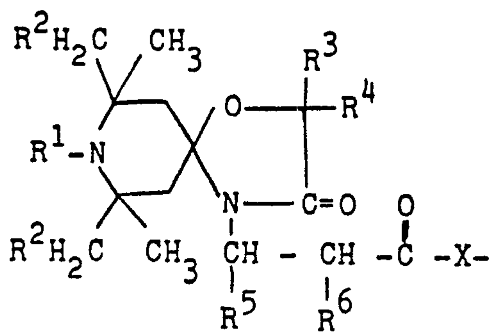

(III),

oder durch $C_1$-$C_{21}$-Alkylcarboxyl, wobei $R^1$, $R^2$, $R^3$, $R^4$, $R^5$, $R^6$ und X die obige Bedeutung haben und $R^8$ Wasserstoff $C_1$-$C_{10}$-Alkyl oder ein Rest

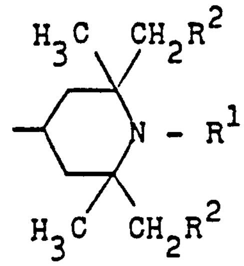

,

worin $R^1$ und $R^2$ die obige Bedeutung haben, ist, bedeutet,

$R^7$ bei n = 2
geradkettiges oder verzweigtes $C_1$-$C_{30}$-Alkylen, $C_2$-$C_{30}$-Alkenylen, Phenyldialkylen, wobei diese Reste durch -O- oder
-N-, worin $R^8$ die obige Bedeutung hat, unterbrochen sein können, bedeutet,
|
$R^8$

$R^7$ bei n = 3 oder 4 einen Rest der Formeln IV, V, VI oder VII,

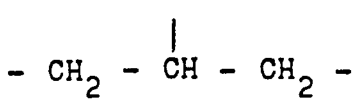

(IV),

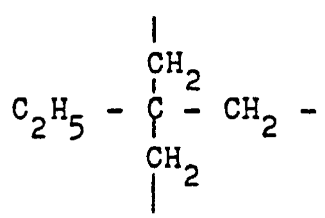

(V),

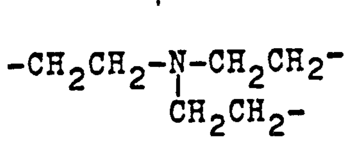

(VI),

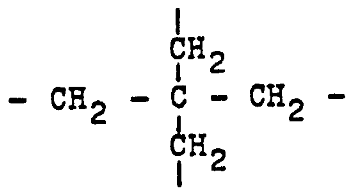 (VII)

bedeutet, und

X -O- oder

$R^8$
|
-N-,

worin $R^8$ die obige Bedeutung hat, ist.

2. Verbindungen nach Anspruch 1, dadurch gekennzeichnet, daß

n 1 oder 2 ist,

$R^1$ Wasserstoff oder Acetyl ist,

$R^2$ Wasserstoff ist,

$R^3$ und $R^4$ unabhängig voneinander $C_1$-$C_7$-Alkyl bedeuten,

$R^5$ und $R^6$ unabhängig voneinander Wasserstoff oder Methyl sind,

$R^7$ $C_2$-$C_{20}$-Alkyl, $C_1$-$C_{30}$-Alkylen, wobei diese durch Reste -O- oder
-N-, unterbrochen sein können,
|
$R^8$

worin $R^8$ Wasserstoff, $C_1$-$C_{10}$-Alkyl oder

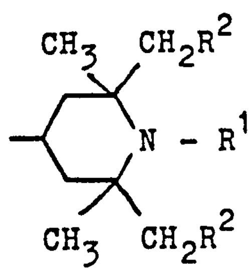

ist, bedeutet, und

X -O- oder
-N-,
|
$R^8$

worin $R^8$ die obige Bedeutung hat, ist.

3. Verfahren zur Herstellung der Verbindungen der Formel I

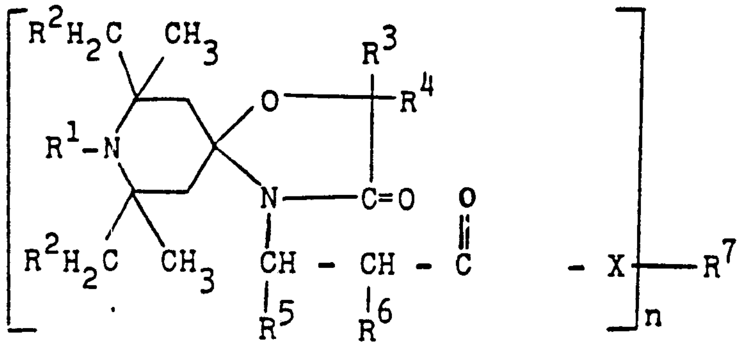

(I),

worin $R^1$, $R^2$, $R^3$, $R^4$, $R^5$, $R^6$, $R^7$, n und X in Anspruch 1 angeführten Bedeutungen haben, durch Umsetzung von 1-Oxa-3-oxo-4,8-diaza-spiro-[4,5]-decan-Verbindungen mit γ-δ-ungesättigten Verbindungen in einem inerten Lösemittel bei einer Temperatur von 30 bis 150°C in Gegenwart eines basischen Katalysators, dadurch gekennzeichnet, daß Verbindungen der Formel VIII

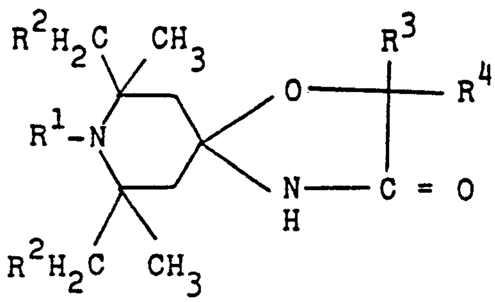

(VIII)

mit Verbindungen der Formel IX

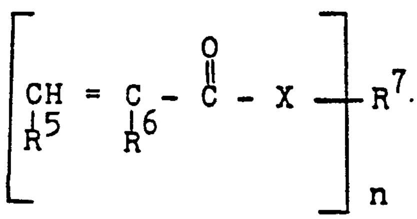

(IX),

wobei $R^1$, $R^2$, $R^3$, $R^4$, $R^5$, $R^6$ und $R^7$ die obengenannte Bedeutung haben, in Gegenwart von 0,05 bis 20 Mol-%, bezogen auf die Verbindung VIII, eines Phasentransferkatalysators in einem bei Raumtemperatur flüssigen aromatischen Kohlenwasserstoff umgesetzt werden.

4. Verfahren nach Anspruch 3, dadurch gekennzeichnet, daß man als Phasentransferkatalysator ein substituiertes Ammonium- oder Phosphoniumsalz oder einen Polyethylenglykoldialkylether verwendet.

5. Verfahren nach Anspruch 4, dadurch gekennzeichnet, daß man als Phasentransferkatalysator ein Tetraalkyl- oder Trialkylbenzylammoniumchlorid verwendet.

6. Verfahren nach Anspruch 4, dadurch gekennzeichnet, daß man als Phasentransferkatalysator ein Tetraalkylphosphoniumbromid verwendet.

7. Verwendung der Verbindungen nach Anspruch 1 - 2 zum Stabilisieren von synthetischen Polymeren.

8. Verwendung der Verbindungen nach Anspruch 1 - 2 zum Stabilisieren von Lacken.

9. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen, 0,01 bis 5 Gewichtateile, bezogen auf Polymeres, eines Stabilisators nach Anspruch 1 - 2 zusetzt.

10. Verfahren zum Stabilisieren von Lacken gegen den schädigenden Einfluß von Licht, dadurch gekennzeichnet, daß man den Lacken, gegebenenfalls neben bisher bekannten, stabilisierend wirkenden Stoffen, 0,02 bis 5 Gewichtsteile, bezogen auf den Lack, eines Stabilisators nach Anspruch 1 - 2 zusetzt.

**Patentansprüche** für den Vertragsstat: AT

1. Verfahren zur Herstellung von 1-Oxa-3-oxo-4,8-diaza-spiro-[4,5]-decan-Verbindungen der Formel I

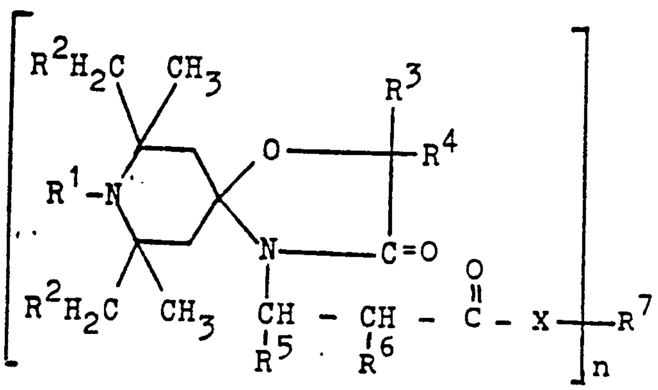

(I),

worin

n eine ganze Zahl von 1 bis 4 ist,

$R^1$ Wasserstoff, $C_1$-$C_4$-Alkyl, Benzyl, Allyl, $C_2$-$C_{30}$-Alkanoyl, $C_3$-$C_{20}$-Alkenoyl, $C_7$-$C_{11}$-Aroyl, $C_8$-$C_{14}$-Arylalkanoyl oder $C_8$-$C_{20}$-Alkylaryl ist,

$R^2$ Wasserstoff oder $C_1$-$C_4$-Alkyl bedeutet,

$R^3$ Wasserstoff, $C_1$-$C_{18}$-Alkyl, $C_5$-$C_{12}$-Cycloalkyl, eine Phenyl- oder Naphthylgruppe, die durch Chlor oder $C_1$-$C_4$-Alkyl substituiert sein kann, oder eine gegebenenfalls durch $C_1$-$C_4$-Alkyl substituierte $C_7$-$C_{12}$-Phenylalkylengruppe ist,

$R^4$ Wasserstoff, $C_1$-$C_4$-Alkyl, $C_5$-$C_{12}$-Cycloalkyl, durch -COOH, Carb-$C_1$-$C_4$-alkoxi oder Carbamoyl substituiertes $C_1$-$C_3$-Alkenyl, eine Phenyl-, Naphthyl- oder Pyridylgruppe, die durch $C_1$-$C_4$-Alkoxi oder $C_1$-$C_4$-Alkyl substituiert sein kann, oder eine $C_7$-$C_{12}$-Phenylalkylgruppe, die durch $C_1$-$C_4$-Alkyl substituiert sein kann, bedeutet, oder

$R^3$ und $R^4$ zusammen mit dem sie bindendem Kohlenstoff eine $C_5$-$C_{12}$-Cycloalkylgruppe, die durch eine bis vier $C_1$-$C_4$-Alkylgruppen substituiert sein kann, oder einen Rest der Formel II

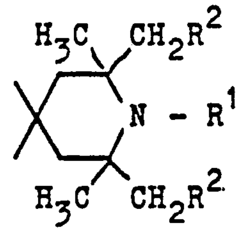

(II),

worin $R^1$ und $R^2$ die obengenannte Bedeutung haben, bilden

$R^5$ Wasserstoff, Methyl, Phenyl oder Carb-$C_1$-$C_{21}$-alkoxi ist,

$R^6$ Wasserstoff oder Methyl bedeutet,

$R^7$ bei n = 1

Wasserstoff, $C_1$-$C_{21}$-Alkyl, $C_2$-$C_{22}$-Alkenyl, $C_7$-$C_{18}$-Phenylalkyl, $C_5$-$C_{12}$-Cycloalkyl, Phenyl, Naphthyl, $C_7$-$C_{18}$-Alkylphenyl, den Rest der Formel

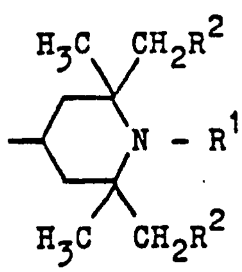

,

worin $R^1$ und $R^2$ die obige Bedeutung haben, $C_2$-$C_{20}$-Alkyl, welches unterbrochen sein kann durch -O- oder

-N- und/oder substituiert
 |
$R^8$

durch einen Rest der Formel III

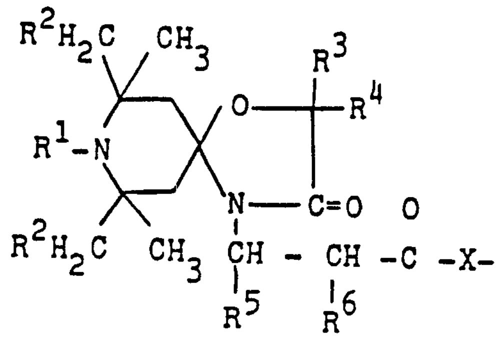

(III),

oder durch $C_1$-$C_{21}$-Alkylcarboxyl, wobei $R^1$, $R^2$, $R^3$, $R^4$, $R^5$, $R^6$ und X die obige Bedeutung haben und $R^8$ Wasserstoff $C_1$-$C_{10}$-Alkyl oder ein Rest

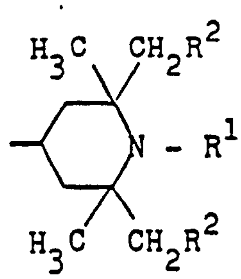

,

worin $R^1$ und R die obige Bedeutung haben, ist, bedeutet,

$R^7$ bei n = 2
geradkettiges oder verzweigtes $C_1$-$C_{30}$-Alkylen, $C_2$-$C_{30}$-Alkenylen, Phenyldialkylen, wobei diese Reste durch -O- oder
-N-,
 |
$R^8$
worin $R^8$ die obige Bedeutung hat, unterbrochen sein können, bedeutet,

$R^7$ bei n = 3 oder 4
einen Rest der Formeln IV, V, VI oder VII,

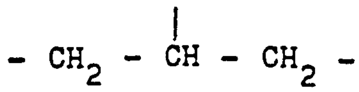

(IV),

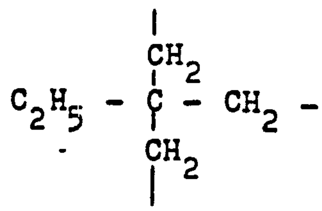

(V),

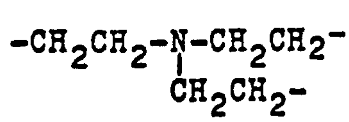

(VI),

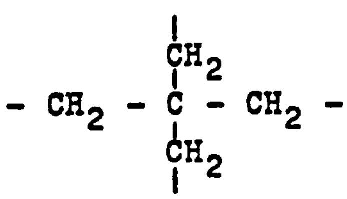

(VII)

bedeutet, und
X -O- oder
R8
|
-N-
worin $R^8$ die obige Bedeutung hat, ist, durch Umsetzung von 1-Oxa-3-oxo-4,8-diaza-spiro-[4,5]-decan-Verbindungen mit γ-δ-ungesättigten Verbindungen in einem inerten Lösemittel bei einer Temperatur von 30 bis 150°C in Gegenwart eines basischen Katalysators, dadurch gekennzeichnet, daß Verbindungen der Formel VIII

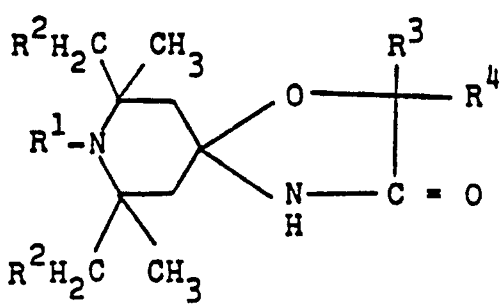

(VIII)

mit Verbindungen der Formel IX

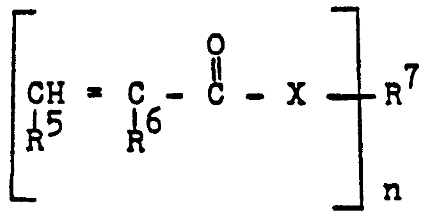

(IX),

wobei $R^1$, $R^2$, $R^3$, $R^4$, $R^5$, $R^6$ und $R^7$ die obengenannte Bedeutung haben, in Gegenwart von 0,05 bis 20 Mol-%, bezogen auf die Verbindung VIII, eines Phasentransferkatalysators in einem bei Raumtemperatur flüssigen aromatischen Kohlenwasserstoff umgesetzt werden.

2. Verfahren nach Anspruch 1, dadurch gekennzeichnet, daß man als Phasentransferkatalysator ein substituiertes Ammonium- oder Phosphoniumsalz oder einen Polyethylenglykoldialkylether verwendet.

3. Verfahren nach Anspruch 2, dadurch gekennzeichnet, daß man als Phasentransferkatalysator ein Tetraalkyl- oder Trialkylbenzylammoniumchlorid verwendet.

4. Verfahren nach Anspruch 2, dadurch gekennzeichnet, daß man als Phasentransferkatalysator ein Tetraalkylphosphoniumbromid verwendet.

5. Verwendung der nach Anspruch 1 hergestellten Verbindungen zum Stabilisieren von synthetischen Polymeren.

6. Verwendung der nach Anspruch 1 hergestellten Verbindungen zum Stabilisieren von Lacken.

7. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen, 0,01 bis 5 Gewichtsteile, bezogen auf Polymeres, eines Stabilisators, hergestellt nach Anspruch 1 - 4, zusetzt.

8. Verfahren zum Stabilisieren von Lacken gegen den schädigenden Einfluß von Licht, dadurch gekennzeichnet, daß man den Lacken, gegebenenfalls neben bisher bekannten, stabilisierend wirkenden Stoffen, 0,02 bis 5 Gewichtsteile, bezogen auf den Lack, eines Stabilisators, hergestellt nach Anspruch 1 - 4, zusetzt.

**Claims** for the contracting states: BE, CH, DE, FR, GB, IT, LI, NL, SR

1. A 1-oxa-3-oxo-4,8-diaza-spiro[4,5]decane compound of the formula

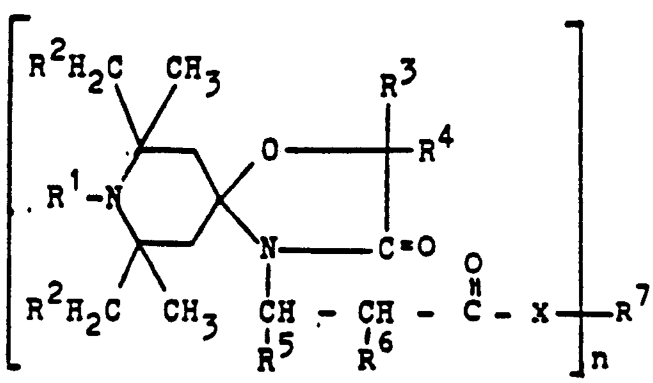

(I)

wherein

n is an integer from 1 to 4,

$R^1$ is hydrogen, $C_1$-$C_4$-alkyl, benzyl, allyl, $C_2$-$C_{30}$-alkanoyl, $C_3$-$C_{20}$-alkenoyl, $C_7$-$C_{11}$-aroyl, $C_8$-$C_{14}$-arylalkanoyl or $C_8$-$C_{20}$-alkylaryl,

$R^2$ is hydrogen or $C_1$-$C_4$-alkyl,

$R^3$ is hydrogen, $C_1$-$C_{18}$-alkyl, $C_5$-$C_{12}$-cycloalkyl, a phenyl or naphthyl group which can be substituted by chlorine or $C_1$-$C_4$-alkyl, or a $C_7$-$C_{12}$-phenylalkylene group which may be unsubstituted or substituted by $C_1$-$C_4$-alkyl,

$R^4$ is hydrogen, $C_1$-$C_4$-alkyl, $C_5$-$C_{12}$-cycloalkyl, $C_1$-$C_3$-alkenyl which is substituted by -COOH, carbo-$C_1$-$C_4$-alkoxy or carbamoyl, a phenyl, naphthyl or pyridyl group which can be substituted by $C_1$-$C_4$-alkoxy or $C_1$-$C_4$-alkyl, or a $C_7$-$C_{12}$-phenylalkyl group which can be substituted by $C_1$-$C_4$-alkyl, or

$R^3$ and $R^4$, together with the carbon atom linking them, form a $C_5$-$C_{12}$-cycloalkyl group, which can be monosubstituted to tetrasubstituted by $C_1$-$C_4$-alkyl groups, or a radical of the formula II

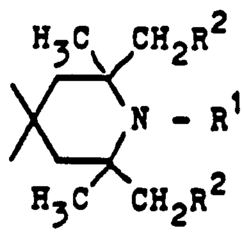

(II)

wherein $R^1$ and $R^2$ are as defined above,

$R^5$ is hydrogen, methyl, phenyl or carbo-$C_1$-$C_{21}$-alkoxy,

$R^6$ is hydrogen or methyl,

$R^7$ is, if n = 1,
hydrogen, $C_1$-$C_{21}$-alkyl, $C_2$-$C_{22}$-alkenyl, $C_7$-$C_{18}$-phenylalkyl, $C_5$-$C_{12}$-cycloalkyl, phenyl, naphthyl, $C_7$-$C_{18}$-alkylphenyl, a radical of the formula

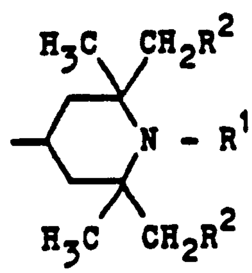

in which
$R^1$ and $R^2$ are as defined above, or $C_2$-$C_{20}$-alkyl which can be interrupted by -O- or
-N
|
$R^8$
and or substituted by a radical of the formula III

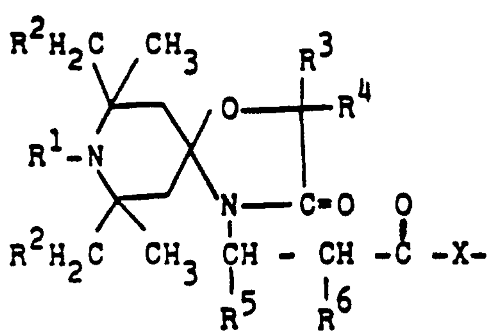

(III)

or by $C_1$-$C_{21}$-alkylcarboxyl, $R^1$, $R^2$, $R^3$, $R^4$, $R^5$, $R^6$ and X being as defined above and

$R^8$ being hydrogen, $C_1$-$C_{10}$-alkyl or a radical

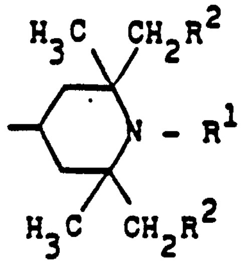

in which

$R^1$ and $R^2$ are as defined above, or

$R^7$ is, if n = 2,

a straight-chain or branch $C_1$-$C_{30}$-alkylene, $C_2$-$C_{30}$-alkenylene or phenyldialkylene, which radicals can be interrupted by -O- or

-N-,
|
$R^8$

$R^8$ being as defined above, or

$R^7$ is, if n = 3 or 4,

a radical of the formulae IV, V, VI or VII

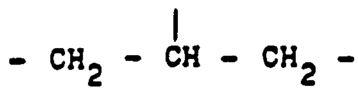

(IV),

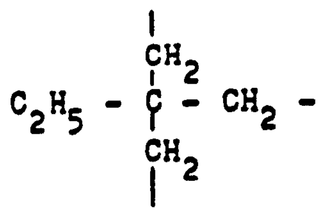

(V),

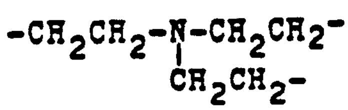

(VI)

(VII)

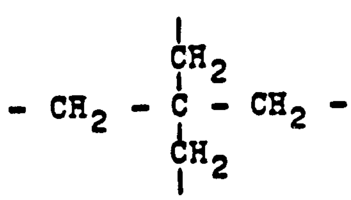

and

X

is -O- or

$R^8$
|
-N-,

$R^8$
being as defined above.

2. A compound as claimed in claim 1, wherein

n is 1 or 2,
$R^1$ is hydrogen or acetyl,
$R^2$ is hydrogen,
$R^3$ and $R^4$ independently of one another are $C_1$-$C_7$-alkyl,
$R^5$ and $R^6$ independently of one another are hydrogen or methyl,
$R^7$ is $C_2$-$C_{20}$-alkyl or $C_1$-$C_{30}$-alkylene, it being possible for these radicals to be interrupted by -O- or -N-,
$\quad|$
$R^8$
$R^8$ being hydrogen, $C_1$-$C_{10}$-alkyl or

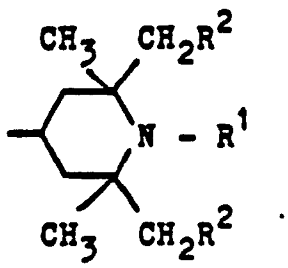

and
X is -O- or -N-,
$\quad|$
$R^8$
$R^8$ being as defined above.

3. A process for the preparation of a compound of the formula I

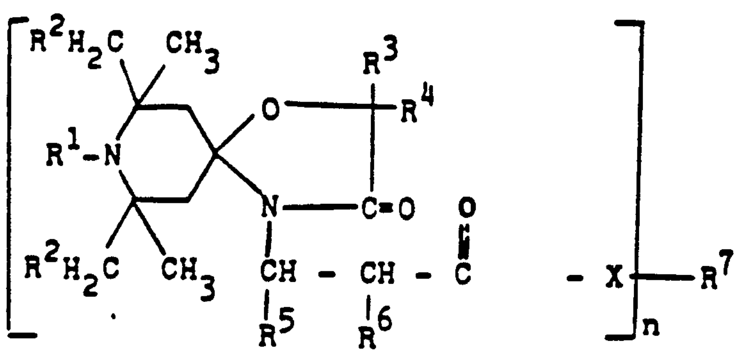

(I),

wherein $R^1$, $R^2$, $R^3$, $R^4$, $R^5$, $R^6$, $R^7$, n and X are as defined in claim 1, by reacting a 1-oxa-3-oxo-4,8-diaza-spiro-[4,5]-decane compound with a γ-δ-unsaturated compound in an inert solvent at a temperature from 30 to 150°C in the presence of a basic catalyst, which comprises reacting a compound of the formula VIII

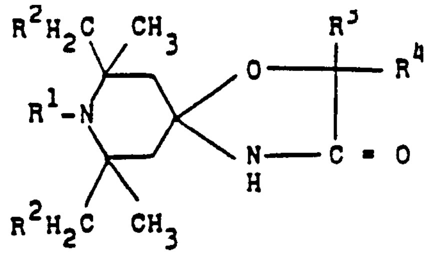

(VIII)

with a compound of the formula IX

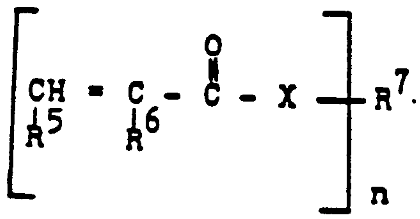

(IX), (IX),

wherein $R^1$, $R^2$, $R^3$, $R^4$, $R^5$, $R^6$ and $R^7$ are as defined above, in the presence of 0.05 to 20 mol-%, relative to the compound VIII, of a phase transfer catalyst in an aromatic hydrocarbon which is liquid at room temperature.

4. The process as claimed in claim 3, wherein the phase transfer catalyst used is a substituted ammonium or phosphonium salt or a polyethylene glycol dialkyl ether.

5. The process as claimed in claim 4, wherein the phase transfer catalyst used is a tetraalkyl- or trialkylbenzylammonium chloride.

6. The process as claimed in claim 4, wherein the phase transfer catalyst used is a tetraalkylphosphonium bromide.

7. The use of a compound as claimed in claim 1 - 2 for stabilizing synthetic polymers.

8. The use of a compound as claimed in claim 1 - 2 for stabilizing surface coatings.

9. A process for stabilizing synthetic polymers against the damaging effect of light, which comprises adding 0.01 to 5 parts by weight, relative to the polymer, of a stabilizer as claimed in claim 1 - 2 to the polymers, if appropriate in addition to previously known substances having a stabilizing action.

10. A process for stabilizing surface coatings against the damaging effect of light, which comprises adding 0.02 to 5 parts by weight, relative to the surface coating, of a stabilizer as claimed in claim 1 - 2 to the surface coatings, if appropriate in addition to previously known substances having a stabilizing action.

**Claims** for the contracting state: AT

1. A process for the preparation of a 1-oxa-3-oxo-4,8-diaza-spiro-[4,5]-decane compound of the formula I

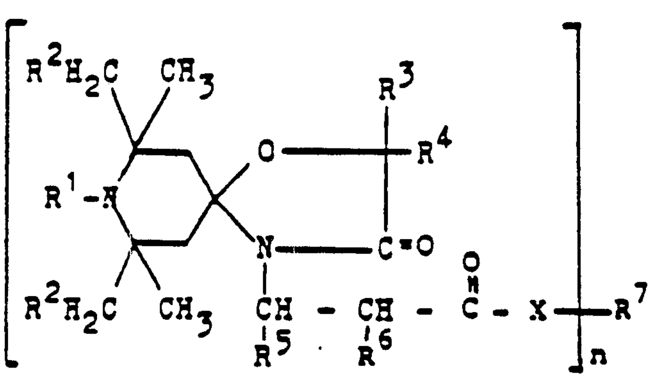

(I),

wherein

n is an integer from 1 to 4,

$R^1$ is hydrogen, $C_1$-$C_4$-alkyl, benzyl, allyl, $C_2$-$C_{30}$-alkanoyl, $C_3$-$C_{20}$-alkenoyl, $C_7$-$C_{11}$-aroyl, $C_8$-$C_{14}$-arylalkanoyl or $C_8$-$C_{20}$-alkylaryl,

$R^2$ is hydrogen or $C_1$-$C_4$-alkyl,

$R^3$ is hydrogen, $C_1$-$C_{18}$-alkyl, $C_5$-$C_{12}$-cycloalkyl, a phenyl or naphthyl group which can be substituted by chlorine or $C_1$-$C_4$-alkyl, or a $C_7$-$C_{12}$-phenylalkylene group which may be unsubstituted or substituted by $C_1$-$C_4$-alkyl,

$R^4$ is hydrogen, $C_1$-$C_4$-alkyl, $C_5$-$C_{12}$-cycloalkyl, $C_1$-$C_3$-alkenyl which is substituted by -COOH, carbo-$C_1$-$C_4$-alkoxy or carbamoyl, a phenyl, naphthyl or pyridyl group which can be substituted by $C_1$-$C_4$-alkoxy or $C_1$-$C_4$-alkyl, or a $C_7$-$C_{12}$-phenylalkyl group which can be substituted by $C_1$-$C_4$-alkyl, or

$R^3$ and

$R^4$, together with the carbon atom linking them, form a $C_5$-$C_{12}$-cycloalkyl group, which can be monosubstituted to tetrasubstituted by $C_1$-$C_4$-alkyl groups, or a radical of the formula II

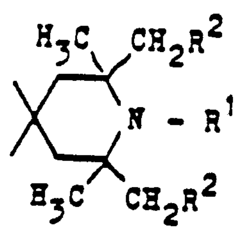

(II)

wherein $R^1$ and $R^2$ are as defined above,

$R^5$ is hydrogen, methyl, phenyl or carb-$C_1$-$C_{21}$-alkoxy,

$R^6$ is hydrogen or methyl,

$R^7$ is, if n = 1,

hydrogen, $C_1$-$C_{21}$-alkyl, $C_2$-$C_{22}$-alkenyl, $C_7$-$C_{18}$-phenylalkyl, $C_5$-$C_{12}$-cycloalkyl, phenyl, naphthyl, $C_7$-$C_{18}$-alkylphenyl, the radical of the formula

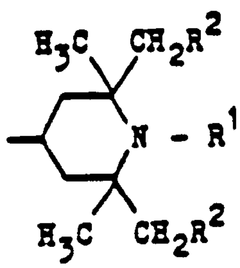

in which

$R^1$ and $R^2$ are as defined above, or $C_2$-$C_{20}$-alkyl which can be interrupted by -O- or

-N

|

$R^8$

and/or substituted by a radical of the formula III

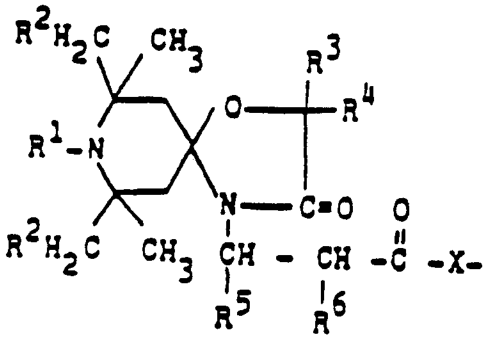

(III)

or by $C_1$-$C_{21}$-alkylcarboxyl $R^1$, $R^2$, $R^3$, $R^4$, $R^5$, $R^6$ and X being as defined above and

$R^8$ being hydrogen, $C_1$-$C_{10}$-alkyl or a radical

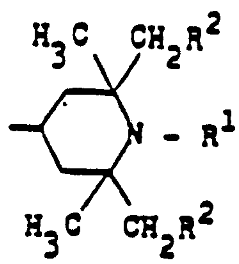

in which

$R^1$ and $R^2$ are as defined above, or

$R^7$ is, if n = 2,

a straight-chain or branched $C_1$-$C_{30}$-alkylene, $C_2$-$C_{30}$-alkenylene or phenyldialkylene, which radicals can be interrupted by -O- or

-N-,
 |
 $R^8$

$R^8$ being as defined above, or

$R^7$ is, if n = 3 or 4,

a radical of the formulae IV, V, VI or VII

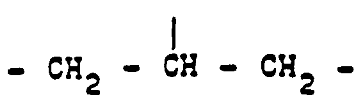

(IV),

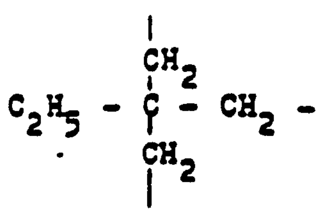

(V),

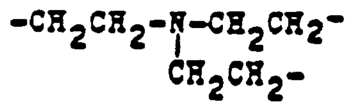

(VI),

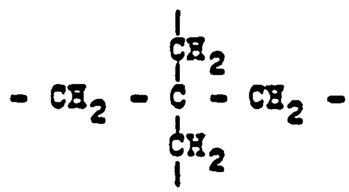

(VII)

and X is -O- or

$R^8$
|
-N-, $R^8$ being as defined above, by reacting a 1-oxa-3-oxo-4,8-diaza-spiro-[4,5]-decane compound with a γ-δ- unsaturated compound in an inert solvent at a temperature from 30 to 150°C in the presence of a basic catalyst, which comprises reacting a compound of the formula VIII

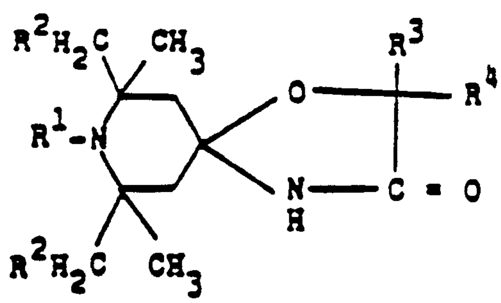

(VIII)

with a compound of the formula IX

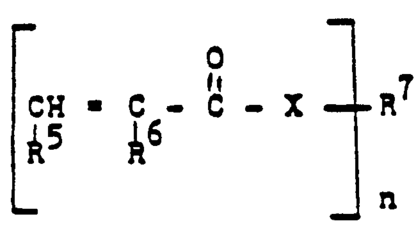

(IX),

wherein $R^1$, $R^2$, $R^3$, $R^4$, $R^5$, $R^6$ and $R^7$ are as defined above, in the presence of 0.05 to 20 mol-%, relative to the compound VIII, of a phase transfer catalyst in an aromatic hydrocarbon which is liquid at room temperature.

2. The process as claimed in claim 1, wherein the phase transfer catalyst used is a substituted ammonium or phosphonium salt or a polyethylene glycol dialkyl ether.

3. The process as claimed in claim 2, wherein the phase transfer catalyst used is a tetraalkyl- or trialkylbenzyl ammonium chloride.

4. The process as claimed in claim 2, wherein the phase transfer catalyst used is a tetraalkylphosphonium bromide.

5. The use of a compound prepared as claimed in claim 1 for stabilizing synthetic polymers.

6. The use of a compound prepared as claimed in claim 1, for stabilizing surface coatings.

7. A process for stabilizing synthetic polymers against the damaging effect of light, which comprises adding 0.01 to 5 parts by weight, relative to the polymer, of a stabilizer prepared as claimed in claim 1 - 4 to the

polymers, if appropriate in addition to previously known substances having a stabilizing action.

8. A process for stabilizing surface coatings against the damaging effect of light, which comprises adding 0.02 to 5 parts by weight, relative to the surface coating, of a stabilizer prepared as claimed in claim 1 - 4 to the surface coatings, if appropriate in addition to previously known substances having a stabilizing action.

**Revendications** pour les Etats Contractants: BE, CH, DE, FR, GB, IT, LI, NL, SE

1. Oxa-1 oxo-3 diaza-4,8 spiro-[4.5]-décanes répondant à la formule I:

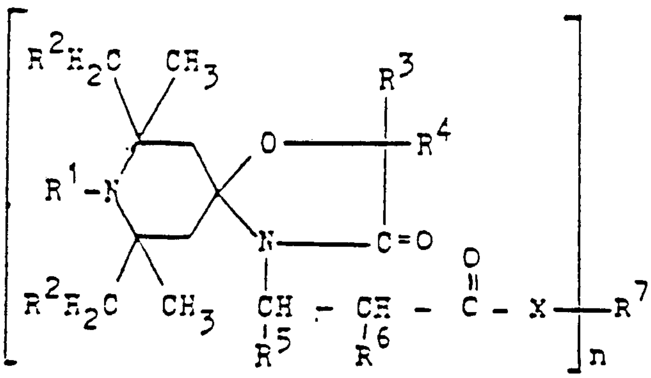

(I)

dans laquelle:

n désigne un nombre entier de 1 à 4,

$R^1$ représente l'hydrogène, un alkyle en $C_1$-$C_4$, un benzyle, un allyle, un alcanoyle en $C_2$-$C_{30}$, un alcénoyle en $C_3$-$C_{20}$, un aroyle en $C_7$-$C_{11}$, un arylalcanoyle en $C_8$-$C_{14}$ ou un alkylaryle en $C_8$-$C_{20}$,

$R^2$ représente l'hydrogène ou un alkylle en $C_1$-$C_4$,

$R^3$ représente l'hydrogène, un alkyle en $C_1$-$C_{18}$, un cycloalkyle en $C_5$-$C_{12}$, un radical phényle ou naphtyle éventuellement porteur d'un atome de chlore ou d'un alkyle en $C_1$-$C_4$, ou un radical phénylalkyle en $C_7$-$C_{12}$ éventuellement porteur d'un alkyle en $C_1$-$C_4$,

$R^4$ représente l'hydrogène, un alkyle en $C_1$-$C_4$, un cycloalkyle en $C_5$-$C_{12}$, un alcényle en $C_1$-$C_3$ porteur d'un -COOH, d'un alcoxycarbonyle à alcoxy en $C_1$-$C_4$ ou d'un carbamoyle, un radical phényle, naphtyle ou pyridyle éventuellement porteur d'un alcoxy en $C_1$-$C_4$ ou d'un alkyle en $C_1$-$C_4$, ou un radical phénylalkyle en $C_7$-$C_{12}$ qui peut porter un alkyle en $C_1$-$C_4$, ou

$R^3$ et $R^4$ forment ensemble, et avec l'atome de carbone qui les unit, un radical cycloalkyle en $C_5$-$C_{12}$ éventuellement porteur d'un à quatre alkyles en $C_1$-$C_4$, ou un radical répondant à la formule II:

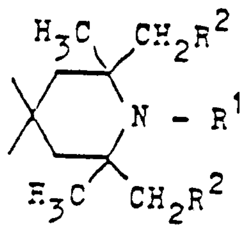

(II)

dans laquelle $R^1$ et $R^2$ ont les significations qui leur ont été données ci-dessus,

$R^5$ représente l'hydrogène, un méthyle, un phényle ou un alcoxycarbonyle à alcoxy en $C_1$-$C_{21}$,

$R^6$ représente l'hydrogène ou un méthyle,

$R^7$ représente, dans le cas où n est égal à 1:
l'hydrogène, un alkyle en $C_1$-$C_{21}$, un alcenyle en $C_2$-$C_{22}$, un phénylalkyle en $C_7$-$C_{18}$, un cycloalkyle en $C_5$-$C_{12}$, un phényle, un naphtyle, un alkylphényle en $C_7$-$C_{18}$, un radical de formule:

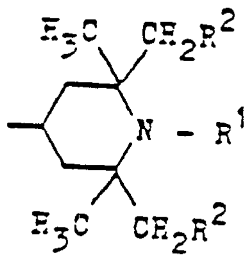

(dans lequel $R^1$ et $R^2$ ont les significations précédemment données), ou un alkyle en $C_2$-$C_{20}$ qui peut être interrompu par -O- ou par

-N
 |
$R^8$

et/ou porter, comme substituant, un radical de formule III:

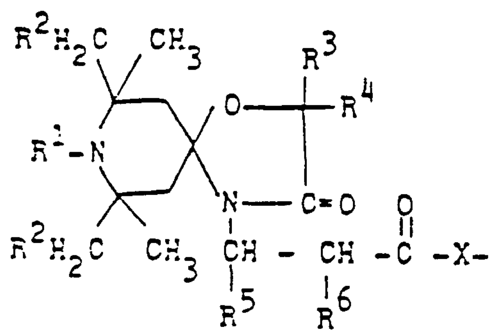

(III)

ou un radical alkylcarbonyle à alkyle en $C_1$-$C_{21}$, les symboles $R^1$, $R^2$, $R^3$, $R^4$, $R^5$, $R^6$ et X ayant les significations indiquées ci-dessus et $R^8$ représentant l'hydrogène, un alkyle en $C_1$-$C_{10}$ ou un radical de formule:

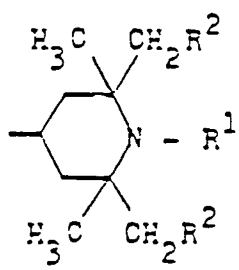

(dans lequel $R^1$ et $R^2$ ont les significations précédemment données),

$R^7$ représente, dans le cas où n est égal à 2:
un alkylène en $C_1$-$C_{30}$ linéaire ou ramifié, un alcénylène en $C_2$-$C_{30}$ ou un phényl-dialkylène, ces radicaux pouvant être interrompus par -O- ou

-N-,
 |
$R^8$

le symbole $R^8$ ayant la signification précédemment donnée,

$R^7$ représente, dans le cas où n est égal à 3 ou 4:
un radical répondant à l'une des formules IV, V, VI et VII:

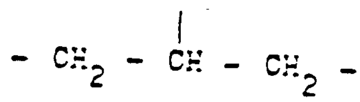 (IV),

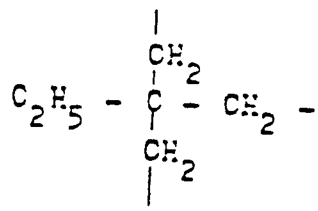 (V),

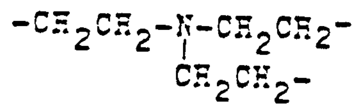 (VI),

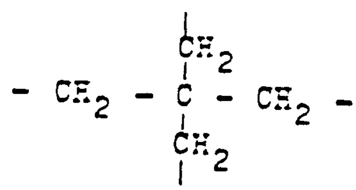 (VII),

et

X représente -O- ou un radical

$R_8$
|
-N

dans lequel $R^8$ a la signification indiquée ci-dessus.

2. Composés selon la revendication 1 caractérisés en ce que:

n est égal à 1 ou à 2,

$R^1$ représente l'hydrogène ou un radical acétyle,

$R^2$ représente l'hydrogène,

$R^3$ et $R^4$ représentent chacun, indépendamment l'un de l'autre, un alkyle en $C_1$-$C_7$,

$R^5$ et $R^6$ représentent chacun, indépendamment l'un de l'autre, l'hydrogène ou un radical méthyle,

$R^7$ représente, un alkyle en $C_2$-$C_{20}$ ou un alkylène en $C_1$-$C_{30}$, ces radicaux pouvant être interrompus chacun par -O- ou par un radical

-N
|
$R_8$

dans lequel $R^8$ désigne l'hydrogène, un alkyle en $C_1$-$C_{10}$ ou un radical de formule:

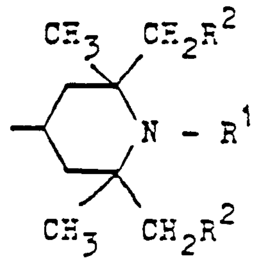

et

X représente -O- ou un radical
-N
|
$R^8$
dans lequel $R^8$ a la signification qui vient de lui être donnée.

3. Procédé pour préparer des composés répondant à la formule I:

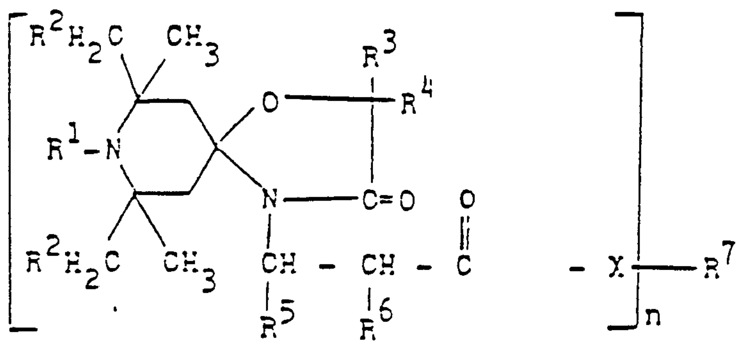

(I)

dans laquelle $R^1$, $R^2$, $R^3$, $R^4$, $R^5$, $R^6$, $R^7$, n et X ont les significations qui leur ont été données à la revendication 1, par réaction d'oxa-1 oxo-3 diaza-4,8 spiro-[4.5]-décanes avec des composés insaturés en $\gamma$, $\delta$, dans un solvant inerte, à une température de 30 à 150°C et en présence d'un catalyseur basique, procédé caractérisé en ce qu'on fait réagir des composés de formule VIII:

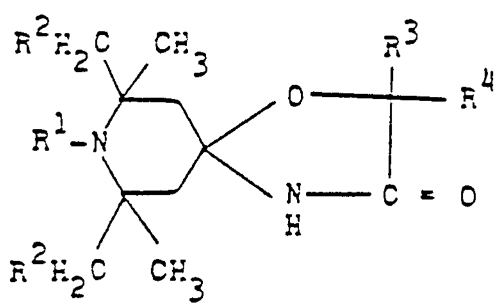

(VIII)

avec des composés de formule IX:

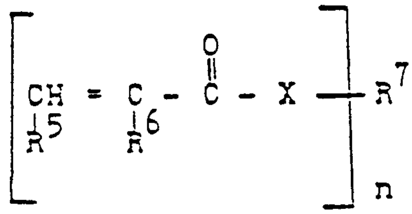

(IX)

formules dans lesquelles $R^1$, $R^2$, $R^3$, $R^4$, $R^5$, $R^6$ et $R^7$ ont les significations qui leur ont été données ci-dessus, en présence de 0,05 à 20 % en moles, par rapport au composé (VIII), d'un catalyseur de transfert de phase, dans un hydrocarbure aromatique qui est liquide à la température ambiante.

4. Procédé selon la revendication 3 caractérisé en ce qu'on utilise, comme catalyseur de transfert de phase, un sel d'ammonium ou de phosphonium substitué ou un éther dialkylique de polyéthylène-glycol.

5. Procédé selon la revendication 4 caractérisé en ce qu'on utilise, comme catalyseur de transfert de phase, un chlorure de tétraalkyl-ammonium ou de trialkyl-benzylammonium.

6. Procédé selon la revendication 4 caractérisé en ce qu'on utilise, comme catalyseur de transfert de phase, un bromure de tétraalkyl-phosphonium.

7. Application des composés selon l'une des revendications 1 et 2 à la stabilisation de polymères synthétiques.

8. Application des composés selon l'une des revendications 1 et 2 à la stabilisation de peintures et de vernis.

9. Procédé pour stabiliser des polymères synthétiques contre les effets destructeurs de la lumière, procédé caractérisé en ce qu'on ajoute aux polymères, éventuellement en plus de composés stabilisants connus, de 0,01 à 5 parties en poids, par rapport au polymère, d'un stabilisant selon l'une des revendications 1 et 2.

10. Procédé pour stabiliser des peintures et des vernis contre les effets destructeurs de la lumière, procédé caractérisé en ce qu'on ajoute aux peintures ou aux vernis, éventuellement en plus de composés stabilisants connus, de 0,02 à 5 parties en poids, par rapport à la peinture ou au vernis, d'un stabilisant selon l'une des revendications 1 et 2.

**Revendications** pour l'Etat contractant: AT

1. Procédé pour préparer des oxa-1-oxo-3-diaza-4,8-spiro-[4.5]-décanes répondant à la formule I:

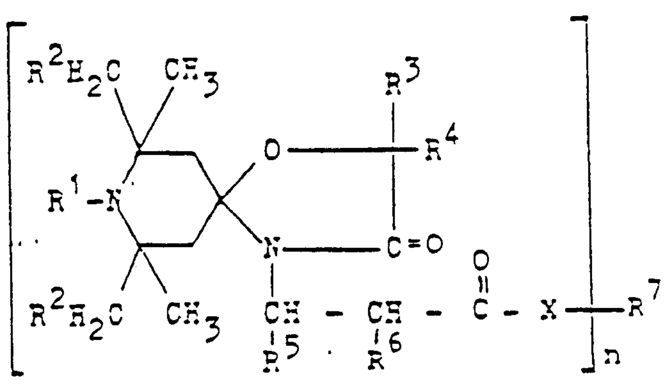

(I),

dans laquelle:

- n désigne un nombre entier de 1 à 4,
- $R^1$ représente l'hydrogène, un alkyle en $C_1$-$C_4$, un benzyle, un allyle, un alcanoyle en $C_2$-$C_{30}$, un alcénoyle en $C_3$-$C_{20}$, un aroyle en $C_7$-$C_{11}$, un arylalcanoyle en $C_8$-$C_{14}$ ou un alkylaryle en $C_8$-$C_{20}$,
- $R^2$ représente l'hydrogène ou un alkyle en $C_1$-$C_4$,
- $R^3$ représente l'hydrogène, un alkyle en $C_1$-$C_{18}$, un cycloalkyle en $C_5$-$C_{12}$, un radical phényle ou naphtyle éventuellement porteur d'un atome de chlore ou d'un alkyle en $C_1$-$C_4$, ou un radical phénylalkyle en $C_7$-$C_{12}$ éventuellement porteur d'un alkyle en $C_1$-$C_4$,
- $R^4$ représente l'hydrogène, un alkyle en $C_1$-$C_4$, un cycloalkyle en $C_5$-$C_{12}$, un alcényle en $C_1$-$C_3$ porteur d'un -COOH, d'un alcoxycarbonyle à alcoxy en $C_1$-$C_4$ ou d'un carbamoyle, un radical phényle, naphtyle ou pyridyle éventuellement porteur d'un alcoxy en $C_1$-$C_4$ ou d'un alkyle en $C_1$-$C_4$, ou un radical phénylalkyle en $C_7$-$C_{12}$ qui peut porter un alkyle en $C_1$-$C_4$, ou

$R^3$ et $R^4$ forment ensemble, et avec l'atome de carbone qui les unit, un radical cycloalkyle en $C_5$-$C_{12}$ éventuellement porteur d'un à quatre alkyles en $C_1$-$C_4$ ou un radical répondant à la formule II:

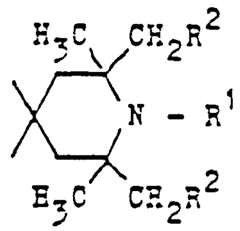

(II)

dans laquelle $R^1$ et $R^2$ ont les significations qui leur ont été données ci-dessus,

$R^5$ représente l'hydrogène, un méthyle, un phényle ou un alcoxycarbonyle à alcoxy en $C_1$-$C_{21}$,

$R^6$ représente l'hydrogène ou un méthyle,

$R^7$ représente, dans le cas où n est égal à 1:

l'hydrogène, un alkyle en $C_1$-$C_{21}$, un alcényle en $C_2$-$C_{22}$, un phénylalkyle en $C_7$-$C_{18}$, un cycloalkyle en $C_5$-$C_{12}$, un phényle, un naphtyle, un alkylphényle en $C_7$-$C_{18}$, un radical de formule:

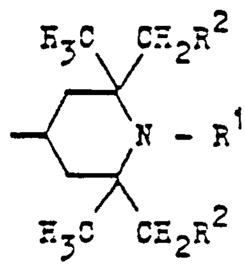

(dans lequel $R^1$ et $R^2$ ont les significations précédemment données), ou un alkyle en $C_2$-$C_{20}$ qui peut être interrompu par -O- ou par

-N- et/ou
 |
$R^8$

et/ou porter, comme substituant, un radical de formule III:

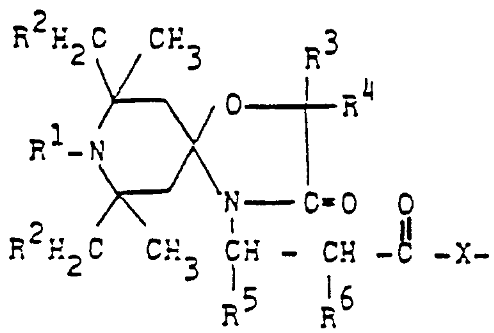

(III)

ou un radical alkylcarbonyle à alkyle en $C_1$-$C_{21}$, les symboles $R^1$, $R^2$, $R^3$, $R^4$, $R^5$, $R^6$ et X ayant les significations indiquées ci-dessus et $R^8$ représentant l'hydrogène, un alkyle en $C_1$-$C_{10}$ ou un radical de formule:

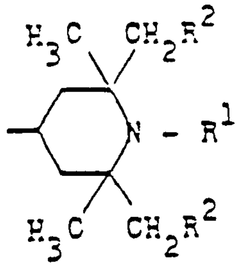

(dans lequel $R^1$ et $R^2$ ont les significations précédemment données),

$R^7$ représente, dans le cas où n est égal à 2:

un alkylène en $C_1$-$C_{30}$ linéaire ou ramifié, un alcénylène en $C_2$-$C_{30}$ ou un phényl-dialkylène, ces radicaux pouvant être interrompus par -O- ou

-N- ,
 |
 $R^8$

le symbole $R^8$ ayant la signification précédemment donnée,

$R^7$ représente, dans le cas où n est égal à 3 ou 4:

un radical répondant à l'une des formules IV, V, VI et VII:

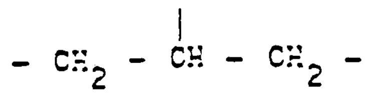

(IV),

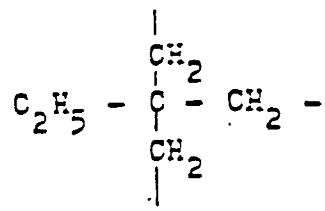

(V),

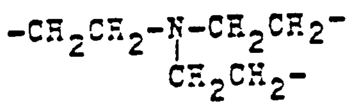

(VI),

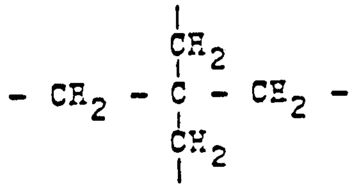

(VII),

et

X représente -O- ou un radical

$R^8$
|
-N

dans lequel $R^8$ a la signification indiquée ci-dessus,

par réaction d'oxa-1 oxo-3 diaza-4,8 spiro-[4.5]-décanes avec des composés insaturés en γ, δ, dans un solvant inerte, à une température de 30 à 150°C et en présence d'un catalyseur basique, procédé caractérisé en ce qu'on fait réagir des composés de formule VIII:

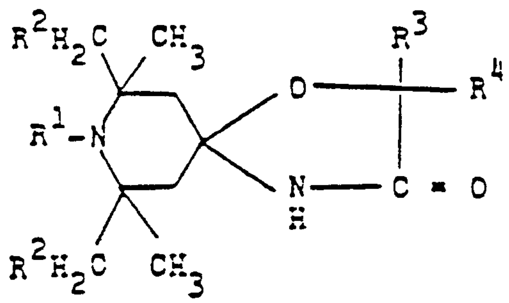

(VIII)

avec des composés de formule IX:

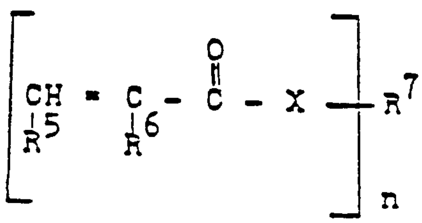

(IX),

formules dans lesquelles $R^1$, $R^2$, $R^3$, $R^4$, $R^5$, $R^6$ et $R^7$ ont les significations qui leur ont été données ci-dessus, en présence de 0,05 à 20 % en moles, par rapport au composé (VIII), d'un catalyseur de transfert de phase, dans un hydrocarbure aromatique qui est liquide à la température ambiante.

2. Procédé selon la revendication 1 caractérisé en ce qu'on utilise, comme catalyseur de transfert de phase, un sel d'ammonium ou de phosphonium substitué ou un éther dialkylique de polyéthylène-glycol.

3. Procédé selon la revendication 2 caractérisé en ce qu'on utilise, comme catalyseur de transfert de phase, un chlorure de tétraalkyl-ammonium ou de trialkyl-benzylammonium.

4. Procédé selon la revendication 2 caractérisé en ce qu'on utilise, comme catalyseur de transfert de phase, un bromure de tétraalkyl-phosphonium.

5. Application des composés préparés selon la revendication 1 à la stabilisation de polymères synthétiques.

6. Application des composés préparés selon la revendication 1 à la stabilisation de peinture et de vernis.

7. Procédé pour stabiliser des polymères synthétiques contre les effets destructeurs de la lumière, procédé caractérisé en ce qu'on ajoute aux polymères, éventuellement en plus de composés stabilisants connus, de 0,01 à 5 parties en poids, par rapport au polymère, d'un stabilisant qui a été préparé selon l'une des revendications 1 à 4.

8. Procédé pour stabiliser des peintures et des vernis contre les effets destructeurs de la lumière, procédé caractérisé en ce qu'on ajoute aux peintures ou aux vernis, éventuellement en plus de composés stabilisants connus, de 0,02 à 5 parties en poids, par rapport à la peinture ou au vernis, d'un stabilisant qui a été préparé selon l'une des revendications 1 à 4.